# EUROPEAN PATENT APPLICATION

(11) **EP 1 169 984 A1**
(43) Date of publication of application: **09.01.2002**
(21) Application number: 01115840.9
(22) Date of filing: 28.06.2001
(51) Int. Cl.: A61F 5/44

(54) **Ostomy bag with integral closure**

(30) Priority: 07.07.2000 GB 0016592
(71) Applicant: SALT & SON LTD., Birmingham B7 4DS (GB)
(72) Inventor: Wiltshire, Neil Philip, Brighton, East Sussex BN1 4AR (GB); Edwards, John Victor, Reigate, Surrey RH2 7HQ (GB)
(74) Representative: Wardley, Diana Mary

(57) **Abstract**

An ostomy bag (10) having a drainage aperture (13) and a sealing device (16) provided for selectively sealing the drainage aperture (13) which is integral with the ostomy bag (10), wherein the integral sealing device (16) includes a first elongate element (17) secured to an outer surface (11a) of a first outer wall (11) of the ostomy bag (10) adjacent the drainage aperture (13) and a second elongate element (18) which is operatively connected to the first elongate element (17), at a first end of each elongate element, by a flexible hinge member (19), and characterised in that the integral sealing device (16) further includes a first closure member (20) at the second end of the first elongate element (17), and a second closure member (21) at the second end of the second elongate element (18), the first and second closure members (20, 21) being engageable to secure the first and second elongate elements (17, 18) together to seal the drainage aperture (13).

## Description

### Description of Invention

This invention relates to an ostomy bag, in particular to an ileostomy bag having a resealable drainage aperture provided by a sealing device which is integral with the bag.

Ostomy bags, such as ileostomy bags, that may be worn by patients for extended periods require periodic draining. Therefore, it is necessary to provide an ostomy bag which enables the user to empty the contents of the bag easily and quickly through a suitable drainage aperture and, once the contents of the bag have been discharged, clean around the drainage aperture and re-seal the drainage aperture so that the bag may continue to be used.

Ostomy bags incorporating resealable drainage apertures are known in the prior art, whereby the drainage aperture is formed between the outer walls of the bag which are otherwise sealed. Typically, the outer walls of the bag are tapered thus forming an elongate portion of the bag adjacent the drainage aperture. In order to effect a temporary seal of the drainage aperture, conventional resealable ostomy bags are typically provided with a separate sealing device, usually some kind of clip or tie which clamps across the elongate bag portion. Such sealing devices rely on the user carefully to roll up the elongate portion and then secure the sealing device in place. Therefore, to empty the bag effectively, the user must completely detach the sealing device from the bag and then roll down the elongate portion.

The conventional separate sealing device arrangement has several inherent disadvantages. First, it is undesirable if a user must, at the same time as emptying the bag into a toilet, hold on to the separate sealing device since there is the possibility that the sealing device may be dropped, possibly into the toilet. A second disadvantage with the separate sealing device arrangement is that it can be awkward for the user to position the sealing device correctly across the rolled-up elongate portion so as to effect a reliable seal. Obviously, any egress of the bag contents through a poorly sealed drainage aperture is most undesirable. A third disadvantage, with some prior art versions of separate sealing devices, is that the separate sealing device is bulky and relatively rigid thus making it uncomfortable for the user should they put pressure on the sealing device pushing it against themselves.

It is an object of the present invention to provide an alternative form of ostomy bag which mitigates the problems described above.

According to a first aspect of the present invention, there is provided an ostomy bag having a drainage aperture and a sealing device provided for selectively sealing the drainage aperture which is integral with the ostomy bag, wherein the integral sealing device includes a first elongate element secured to an outer surface of a first outer wall of the ostomy bag adjacent the drainage aperture and a second elongate element which is operatively connected to the first elongate element, at a first end of each elongate element, by a flexible hinge member, and wherein the integral sealing device further includes a first closure member at the second end of the first elongate element, and a second closure member at the second end of the second elongate element, the first and second closure members being engageable to secure the first and second elongate elements together to seal the drainage aperture.

It can readily be seen how the ostomy bag of the invention overcomes the first and second of the disadvantages described above. Clearly the sealing device cannot be dropped as it is secured to the bag, but also it cannot be incorrectly aligned when being used to seal the bag.

Preferably first and second closure members are offset from the planes of the first and second elongate elements and provide oppositely directed slots such that they are engageable to secure the first and second elongate elements together to seal the drainage aperture.

The first and second closure members preferably further provide, on their faces which are in contact when they are engaged, retaining elements to retain the closure members in engagement.

The retaining elements are conveniently oppositely directed steps on the faces in contact, and ramp portions are provided to enable the faces to ride over each other such that the closure members can engage.

Preferably the sealing device further includes a formation on the second elongate member to assist in disengaging the first and second closure members when they are engaged.

The flexible hinge member preferably enables the second elongate member to move relative to the first elongate member with several degrees of freedom and is conveniently formed from a plastics material.

Preferably the ostomy bag further comprises a resilient member secured to an outer surface of a second outer wall of the ostomy bag adjacent the drainage aperture, and curved so as to tend to open the drainage aperture.

Conveniently the resilient member is aligned with the first elongate member, and when the sealing device is sealing the drainage aperture it is held within the sealing device and flatten such that it cannot open the drainage aperture.

The resilient member may be formed from plastics material.

An embodiment of the invention will now be described, by way of example only, with reference to the accompanying drawings in which:
FIGURE 1 is a view of the rear of an ostomy bag according to the invention when the drainage aperture is not sealed;
FIGURE 2 is a side view of the ostomy bag of Figure 1 also when the drainage aperture is not sealed;

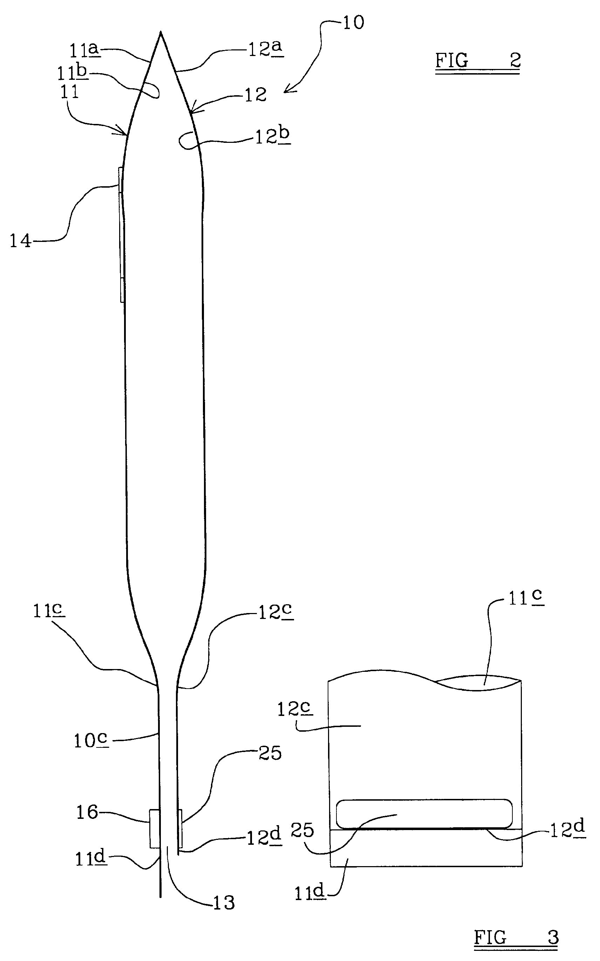

FIGURE 3 is a partial view of the front of the bag of Figures 1 and 2 again when the drainage aperture is not sealed;

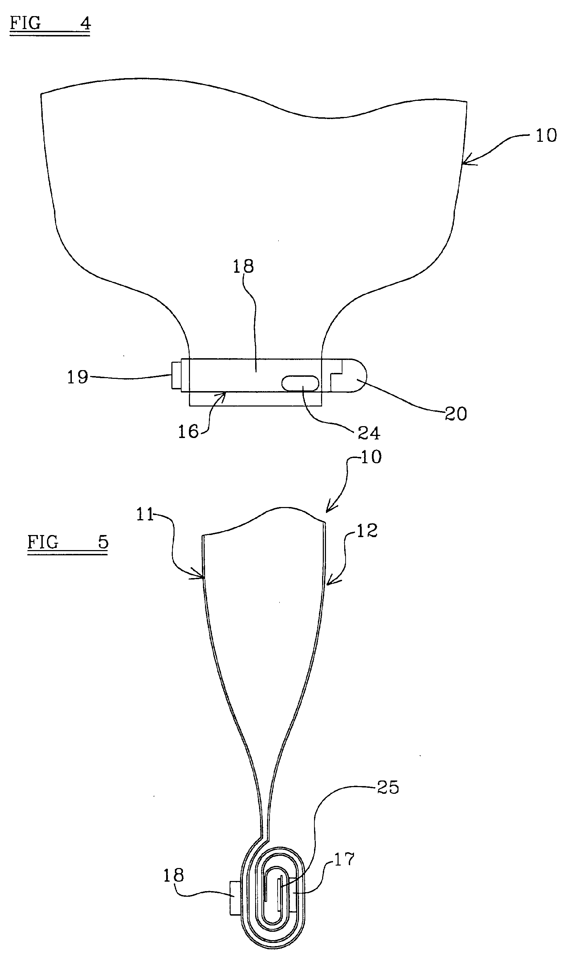

FIGURE 4 is a partial rear view of the bag of Figures 1 to 3 with the drainage aperture sealed;
FIGURE 5 is a partial side view of the bag of Figures 1 to 3 again with the drainage aperture sealed;
FIGURE 6 provides views of the sealing device of the ostomy bag, and
FIGURE 7 provides views of the resilient member of the ostomy bag.

Referring to the Figures an ostomy bag 10 has a first outer wall 11 with outer and inner surfaces 11a and 11b respectively, and a second outer wall 12 with outer and inner surfaces 12a and 12b respectively. The first and second outer walls 11, 12 are sealed around the majority of their outer edges to form the bag 10, but have elongate portions 11c and 12c respectively which extend downwardly at the bottom of the bag 10 to form an elongate portion 10c of the bag 10. The sides of the elongate portions 11c and 12c are sealed but the bottom edges 11d and 12d are not and thus form a drainage aperture 13. The elongate portion 11c of the first outer wall 11 is longer than that of the second outer wall 12, for reasons which will be explained below.

The bag 10 is provided in known manner with a securing ring 14 for attaching the bag to the wearer, and an inlet aperture 15 surrounded by the securing ring 14. The bag 10 may have additional intermediate walls, filters etc. as are known in the prior art, but as they are not relevant to the present invention will not be described here.

The bag 10 further includes a sealing device 16 which has a first elongate element 17 secured to the outer surface 11a of the first outer wall 11 adjacent the drainage aperture 13, and substantially parallel to the bottom 11d of the first outer wall 11. It also has a second elongate element 18 which is operatively connected to the first elongate element 17, at first ends of each of the elongate elements 17, 18, by a flexible hinge member 19. A first closure member 20 is provided at the second end of the first elongate element 17, and a second closure member 21 is provided at the second end of the second elongate element 18. The first and second closure members 20, 21, are engageable to seal the drainage aperture 13 as will be described below.

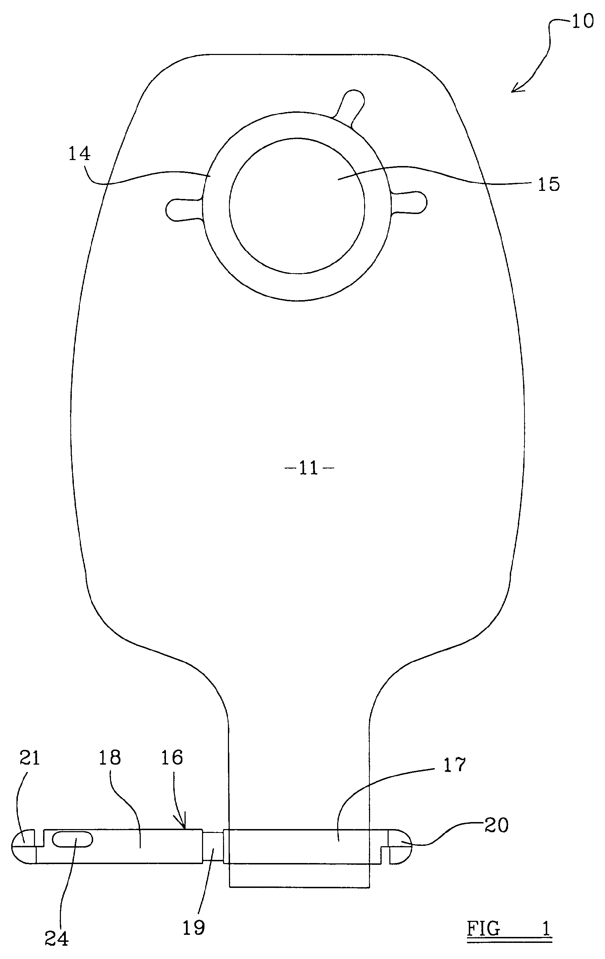

The hinge member 19 is sufficiently flexible to permit movement of the second elongate element 18 relative to the first elongate element 17 with several degrees of freedom. That is, not only can the second elongate element 18 be brought through 180 degrees from the position shown in Figure 1 to lie parallel to the first elongate element 17, but it can be moved out of alignment with the first elongate element 17 to enable the first and second closure members 20, 21 to be engaged with each other as will now be described.

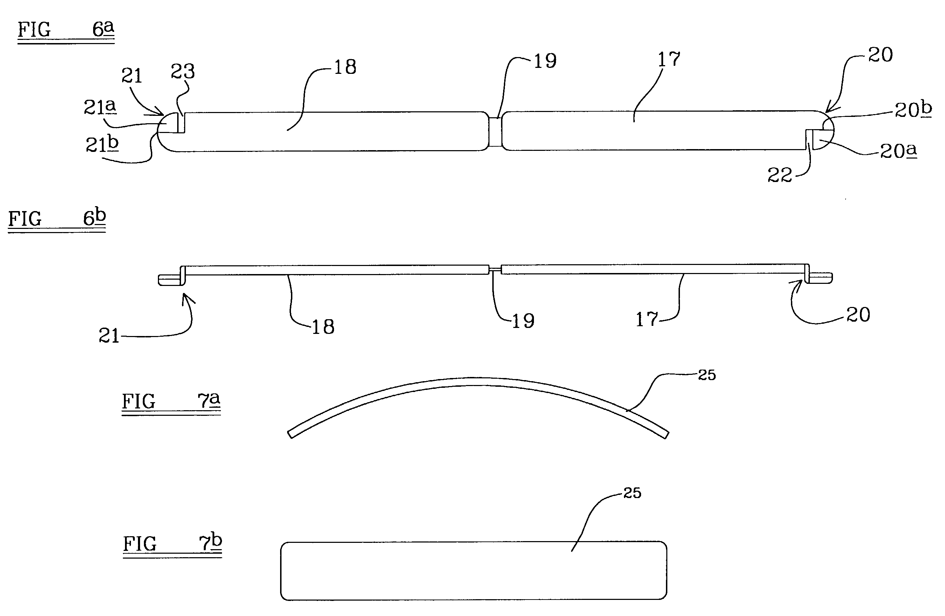

The first closure member 20, as best seen in Figure 6, is connected to the first elongate element 17 across half it's width, thus providing a slot 22, and is offset from the plane of the first elongate element 17 by the thickness of the element 17. Likewise, the second closure member 21 is connected to the second elongate element 18 across half it's width, thus providing a slot 23, and is offset from the plane of the second elongate element 18 by the thickness of the element 18. The slots 22 and 23 face in opposite directions, such that when the second elongate element 18 is brought round to lie alongside the first elongate element 17, and moved slightly off line the slots 22 and 23 can be engaged with each other.

The first and second closure members 20, 21 further provide features to retain the sealing device in it's closed condition as will now be described. The faces of the closure members 20, 21 which come into contact when engaged with each other each comprise a ramp portion 20a and 21a respectively adjacent the respective slot 22, 23, and a step 20b and 21b respectively. Thus when the first and second closure members 20, 21 are engaged the ramp portions 20a and 21a ride over each other forcing the closure members 20, 21 slightly apart, and then the steps 20b and 21b engage with each other to retain the sealing device 16 closed.

The sealing device 16 has a recess 24 on the second elongate element 18 adjacent the second closure member 21. This is provided to assist the wearer in undoing the sealing device 16 when it is closed and they wish to empty the bag 10. The recess 24 is located such that a light pressure on the recess 24 readily disengages the steps 20b and 21b from each other allowing the closure members 20, 21 to disengage and the second elongate element 18 to be moved to the position shown in figure 1.

The bag 10 further includes a resilient member 25 secured to the outer surface 12a of the elongate portion 12c of the second outer wall 12 adjacent the drainage aperture 13. The resilient member 25 is curved, such that it tends to pull the second elongate portion 12c away from the first elongate portion 11c at their bottom ends 11d and 12d to open the drainage aperture 13. This does assist in emptying the bag 10 but is primarily provided to assist the wearer in cleaning around the drainage aperture 13 after the bag 10 has been emptied, but before it is resealed. The extra length of the elongate portion 11c of the first outer wall 11 is also provided to assist the wearer in cleaning around the drainage aperture 13, as it is easier to separate the two ends 11d and 12d when they are not aligned with each other.

The resilient member 25 is in line with the sealing device 16 and is thus secured between the first and second elongate elements 17, 18 when the sealing device 16 is closed, thus bringing it into a flat configuration when the bag 10 is sealed.

The sealing device 16 is used as follows to seal the bag 10. The elongate portion 10 c of the bag 10 is rolled up around the first elongate element 17 until the sealing device 16 is close to the body of the bag 10. The second elongate element 18 is then brought round into line with the first elongate element 17 and the first and second closure members engaged to close the sealing device 16.

To unseal the bag 10 the above described process is reversed. The wearer puts light pressure on the recess 24 on the second elongate element 18, disengages the closure members 20, 21 and moves the second elongate element 18 to the open position and unrolls the elongate portion 10c of the bag 10 thus opening the drainage aperture 13.

The sealing device 16 may conveniently be moulded from a plastics material such as polypropylene copolymer and welded to the bag 10. However, the sealing device 16 may be formed by any other method, from any other material, and secured to the bag by any other means, as appropriate.

The resilient element 25 is conveniently made from a plastics material such as acetyl copolymer, and welded to the bag 10, but may also be formed from any appropriate material and secured to the bag by any appropriate means.

In the present specification "comprise" means "includes or consists of and "comprising" means "including or consisting of".

The bag 10 is described as incorporating a particular method of attachment to the weaver. However, any appropriate method of attachment may be incorporated into an ostomy bag in accordance with the invention.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

## Claims

1. An ostomy bag (10) having a drainage aperture (13) and a sealing device (16) provided for selectively sealing the drainage aperture (13) which is integral with the ostomy bag (10), wherein the integral sealing device (16) includes a first elongate element (17) secured to an outer surface (11a) of a first outer wall (11) of the ostomy bag (10) adjacent the drainage aperture (13) and a second elongate element (18) which is operatively connected to the first elongate element (17), at a first end of each elongate element, by a flexible hinge member (19), and **characterised in that** the integral sealing device (16) further includes a first closure member (20) at the second end of the first elongate element (17), and a second closure member (21) at the second end of the second elongate element (18), the first and second closure members (20, 21) being engageable to secure the first and second elongate elements (17, 18) together to seal the drainage aperture (13).

2. An ostomy bag (10) according to claim 1 **characterised in that** the first and second closure members (20, 21) are offset from the planes of the first and second elongate elements (17, 18) and provide oppositely directed slots (22, 23) such that they are engageable to secure the first and second elongate elements (17, 18) together to seal the drainage aperture (13).

3. An ostomy bag (10) according to claim 2 **characterised in that** the first and second closure members (20, 21) further provide, on their faces which are in contact when they are engaged, retaining elements (20a, 20b, 21a, 21b) to retain the closure members (20, 21) in engagement.

4. An ostomy bag (10) according to claim 3 characterised the retaining elements are oppositely directed steps (20b, 21b) on the faces in contact, and ramp portions (20a, 21a) are provided to enable the faces to ride over each other such that the closure members (20, 21) can engage.

5. An ostomy bag (10) according to according to claims 1 to 4 **characterised in that** the sealing device (16) further includes a formation (24) on the second elongate member (18) to assist in disengaging the first and second closure members (20, 21) when they are engaged.

6. An ostomy bag (10) according to claims 1 to 5 **characterised in that** the flexible hinge member (19) enables the second elongate member (18) to move relative to the first elongate member (17) with several degrees of freedom.

7. An ostomy bag (10) according to any one of the preceding claims **characterised in that** the sealing device (16) is formed from a plastics material.

8. An ostomy bag (10) according to any one of the preceding claims **characterised in that** it further comprises a resilient member (25) secured to an outer surface (12a) of a second outer wall (12) of the ostomy bag (10) adjacent the drainage aperture (13), and curved so as to tend to open the drainage aperture (13).

9. An ostomy bag (10) according to claim 8 as dependent upon any one of claims 1 to 7 **characterised in that** wherein the resilient member (25) is aligned with the first elongate member (17), and when the sealing device (16) is sealing the drainage aperture (13) it is held within the sealing device (16) and flatten such that it cannot open the drainage aperture (13).

10. An ostomy bag (10) according to claim 8 or 9 **characterised in that** the resilient member (25) is formed from plastics material.
